# EUROPEAN PATENT APPLICATION

(11) **EP 3 144 833 A1**
(43) Date of publication of application: **22.03.2017**
(21) Application number: 16169983.0
(22) Date of filing: 17.05.2016
(51) Int. Cl.: G06F 19/00

(54) **COMPUTER APPARATUS AND METHOD FOR ENHANCING CLINICAL PATHWAYS**

(30) Priority: 16.09.2015 GB 201516433
(71) Applicant: FUJITSU LIMITED, 211-8588 Kanagawa (JP)
(72) Inventor: HU, Bo, Winchester, Hampshire SO23 7DT (GB); NASEER BUTT, Aisha, Hayes, Middlesex UB4 8LB (GB)
(74) Representative: Fenlon, Christine Lesley

(57) **Abstract**

A computer-implemented clinical pathway enhancement method and apparatus for enhancing a clinical pathway having a plurality of care steps, where the clinical pathway is a predefined clinical pathway which is associated with an individual patient or a cohort of patients, or a merged clinical pathway obtained by merging two or more such predefined clinical pathways associated with the patient or patient cohort, is proposed in which the clinical pathway is integrated with at least one non-clinical process so as to associate non-clinical data from the non-clinical process with at least one care step which is related to that non-clinical data; and using the non-clinical data associated with the care step to produce one or more enriched clinical pathways. According to one or more predefined refinement conditions, it may also be determined to split or merge one or more care steps of the enriched clinical pathway to produce a refined enriched clinical pathway. Additionally and/or alternatively, where the total number of enriched clinical pathways is greater than one, status conditions indicative of the current status of the patient may be used to dynamically select a preferred clinical pathway which best meets one or more preset criteria. Predefined clinical pathways may be merged to form a merged clinical pathway for enhancement in the clinical pathway enhancement method/apparatus by comparing the care steps and step order in one of the predefined clinical pathways with the care steps and step order in one or more other predefined clinical pathways, identifying any care steps in the one predefined clinical pathway which match care steps and step order in one or more of the other predefined clinical pathways, selecting from any unmatched care steps in the predefined clinical pathways one or more care steps which satisfies most appropriately requirements for the merged clinical pathway which take into account the matched care steps, step order and information associated with the unmatched care step(s), and forming the merged clinical pathway from the matching care steps and the selected care step(s).

## Description

### Background of the Invention

The present invention relates to computer apparatus and a method for enhancing clinical pathways.

Clinical authorities normally draw expertise from all concerned domains and compose and publish clinical guidelines as to the recommended best practice for practitioners to follow. These guidelines are implemented as instances of clinical pathways based on the constitution of the particular situation that each case presents. Decisions on how to treat patients using the clinical pathways are likely to require information from multidisciplinary resources and materials, e.g. administrative, personnel, education level, resource availability and other operational data.

A modern healthcare system is normally formed from a complex network of interacting actors, processes and sub-systems. With the advance of genetic and imaging technologies and the fast expansion of clinical knowledge, it is increasingly likely that more than one actor (for example, several clinicians, in more than one hospital or clinical centre, in the same or different countries) can be involved in the treatment of a particular patient. This imposes an unprecedented challenge to computer-based healthcare systems: instead of dealing with one established pathway, a patient may be subject to a raft of clinical pathways (e.g. exercised by different clinical centres). Moreover, human beings seldom follow the text-book symptom description. Very frequently, a patient is presented to a health centre with more than one disease (co-morbidity), In case of co-morbidity, clinical pathways for different diseases need to be integrated and assessed so the clinicians can identify the best investigation and treatment solutions.

In practice, clinical pathways may suffer from the following inefficiencies which can lead to sub-optimum treatment for a patient and/or increased healthcare costs to individuals and organisations (as well as national healthcare systems),
1. Though not at the same level as national or regional standards, clinical pathways are normally subject to extensive discussion and agreement. An advantage of a predefined clinical pathway is that it should remove undesired variations in treatment, and any consequent undesired legal and financial consequences. However, the generality aspect of a clinical pathway can make it less applicable and adaptable against each individual patient or cohort of patients, as widely applicable pathways may not be suitable for an individual patient or patient cohort.
2. In practice, clinical pathways are normally reinforced on a case-by-case basis and are monitored through means other than computer-based automation. This tends to increase the cost and/or lead to undesired variation owing to human error or preferences on the one hand, while making the entire process less transparent.
3. Pathways can be considered as a path of scheduled care with respect to patients with common diseases. In practice, however, patients normally present multiple disorders (co-morbidity). Those involved in their care are therefore facing the challenge of integrating multiple different predefined clinical pathways. Such integration has to be done on an ad hoc basis, based on a patient's individual needs.
4. Pathways normally focus on clinical conditions and outcomes. In practice, however, there are often non-clinical processes (such as business processes in an organisation) which can influence or conflict with the clinical pathways, and therefore need to be considered alongside the predefined clinical pathway to achieve the best outcomes and to provide patients with the best treatments.

### Brief Summary of the Invention

According to an embodiment of a first aspect of the present invention there is provided computer apparatus configured to carry out a clinical pathway enhancement method in respect of a clinical pathway having a plurality of care steps, where the clinical pathway is a predefined clinical pathway which is associated with an individual patient or a cohort of patients, or a merged clinical pathway obtained by merging two or more such predefined clinical pathways associated with the patient or patient cohort, the apparatus comprising: integration means configured to integrate the clinical pathway with at least one non-clinical process, the integration means being operable to associate non-clinical data from the non-clinical process with at least one care step which is related to that non-clinical data: and enriched pathway formation means configured to use the non-clinical data associated with the care step to produce one or more enriched clinical pathways.

The enriched pathway formation means may be operable to align one or more predefined criteria of care steps in the enriched clinical pathway with one or more predefined criteria of related non-clinical steps in the non-clinical process

The integration means may be operable to associate with a care step non-clinical data relating to administrative and/or clinical procedures, and/or clinical assets of one or more clinical centres, where the administrative and/or clinical procedures comprise at least one of: authorisation requirements; timing requirements; personnel requirements; cost requirements; and equipment requirements; and where the clinical assets comprise at least one of: staff resources; facilities; location; and equipment resources.

Apparatus embodying the first aspect of the present invention may further comprise refinement means configured to determine, according to one or more predefined refinement conditions, whether to split or merge one or more care steps of the enriched clinical pathway, and, if so determined, to split or merge the care step(s) to produce a refined enriched clinical pathway. The refinement conditions may comprise at least one of: an expected positive or negative change in the patient's biological markers at that care step beyond a first predetermined threshold; an expected positive or negative change in the financial cost of the care step beyond a second predetermined threshold; and a requirement for that care step to involve more than one clinical centre.

Apparatus embodying the first aspect of the present invention may further comprise selection means operable, where the total number of enriched clinical pathways is greater than one, to use status conditions indicative of the current status of the patient to dynamically select a preferred clinical pathway, from the enriched clinical pathways, which best meets one or more preset criteria. The selection means may be operable to select the preferred clinical pathway by identifying a set of candidate care steps from all the care steps in the enriched clinical pathways and ranking the candidate steps in the set in accordance with one or more preset criteria, and may identify the candidate care steps by comparing input conditions of each care step with a patient's current status. The one or more preset criteria for ranking the candidate steps may comprise at least one of: degree of similarity between the current patient status and the input conditions of the care step; likely output conditions of the care step; and possible prognosis as a result of the care step.

Apparatus embodying the first aspect of the present invention may be operable, before carrying out the enrichment method, to model the or each predefined clinical pathway as a graph having labelled vertices respectively representing different care steps in the clinical pathway and interconnecting lines between the vertices representing the flow of the pathway, wherein the graph is subsequently used in the enrichment method.

Apparatus embodying the first aspect of the present invention may further comprise pathway merging means configured to merge predefined clinical pathways to form a merged clinical pathway for enrichment in the clinical pathway enhancement method, which pathway merging means are operable to: compare the care steps and step order in one of the predefined clinical pathways with the care steps and step order in one or more other predefined clinical pathways; identify any care steps in the one predefined clinical pathway which match care steps and step order in one or more of the other predefined clinical pathways; select from any unmatched care steps in the predefined clinical pathways one or more care steps which satisfies most appropriately requirements for the merged clinical pathway which take into account the matched care steps, step order and information associated with the unmatched care step(s); and form the merged clinical pathway from the matching care steps and the selected care step(s).

According to an embodiment of a second aspect of the present invention there is provided a computer-implemented clinical pathway enhancement method for enhancing a clinical pathway having a plurality of care steps, where the clinical pathway is a predefined clinical pathway which is associated with an individual patient or a cohort of patients, or a merged clinical pathway obtained by merging two or more such predefined clinical pathways associated with the patient or patient cohort, the method comprising: integrating the clinical pathway with at least one non-clinical process so as to associate non-clinical data from the non-clinical process with at least one care step which is related to that non-clinical data; and using the non-clinical data associated with the care step to produce one or more enriched clinical pathways.

A method embodying the second aspect of the present invention may further comprise determining, according to one or more predefined refinement conditions, whether to split or merge one or more care steps of the enriched clinical pathway, and, if so determined, splitting or merging the care step(s) to produce a refined enriched clinical pathway.

A method embodying the second aspect of the present invention may further comprise using status conditions indicative of the current status of the patient to dynamically select a preferred clinical pathway, where the total number of enriched clinical pathways is greater than one, which best meets one or more preset criteria.

According to an embodiment of a third aspect of the present invention there is provided a computer program which, when run on a computer, causes that computer to carry out a method embodying the second aspect of the present invention.

Apparatus and methods embodying the present invention can enrich and align clinical pathways with non-clinical processes to incorporate non-clinical data, to assist in decision making and ensure the most suitable services are delivered to a patient or a cohort of patients at the right moment.

Embodiments of the invention may additionally:
1. Formulate new pathways by merging multiple clinical pathways in real-time to deal with complex disorders (i.e. a co-presence of multiple disorders), for example by using graph-based and similarity propagation-based pathway integration. The system can leverage critical paths in pathway integration and recommend the best integrated solution for a patient;
2. Refine pathways to define clear milestones and checkpoints to avoid delay in treatment;
3. Adapt clinical pathways based on the changing condition of a patient or a cohort of patients. The system preferably takes into account multiple sources of information for adapting a particular pathway; and/or
4. Adapt pathways according to individual situations of patients to achieve an optimum solution

### Brief Description of the Drawings

Figure 1 is a flowchart illustrating a clinical pathway enhancement method embodying the present invention;
Figure 2 is a flowchart illustrating an optional pathway merging process for use in the method of Figure 1;
Figure 3 is a flowchart illustrating an example of an enrichment process for use in the method of Figure 1;
Figure 4 is a flowchart illustrating an example of a selection process for use in the method of Figure 1;
Figure 5 is a block diagram of apparatus embodying the present invention; and
Figure 6 is a block diagram of a computing device suitable for carrying out a method embodying the present invention.

### Detailed Description of the Invention

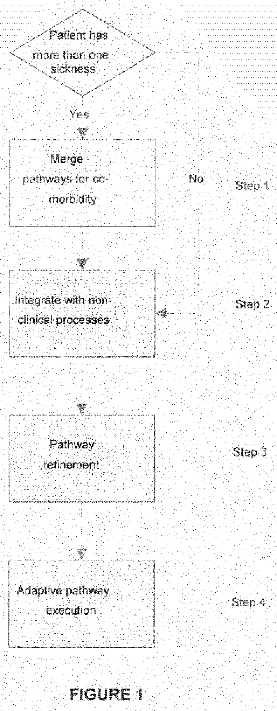

A flowchart of a clinical pathway enhancement method embodying the present invention is shown in Figure 1. The illustrated method is described with reference to an individual patient and comprises an optional first step (Step 1) of merging predefined clinical pathways for co-morbidity if a patient has more than one illness to which a predefined clinical pathway has been assigned. After the clinical pathways have been merged, or if there is only one relevant predefined clinical pathway, the second step (Step 2) of the method comprises integrating the clinical pathway with data from one or more non-clinical processes, from which an enriched clinical pathway is produced. Then, in an optional third step (Step 3) of the method, the enriched clinical pathway may be refined so as to split the clinical pathway at one or more care steps, so as to produce two or more enriched clinical pathways. In a further optional fourth step (Step 4) the enriched clinical pathways may be adapted to the current status of the patient and at least one preferred enriched clinical pathway selected.

In order to deal effectively with a patient having multiple disorders, an embodiment of the present system merges two or more clinical pathways into a single pathway for better alignment and information transparency. In Step 1 of the method of Figure 1, where the patient has more than one medical condition that requires treatment, a preliminary process is carried out in order to merge the predefined clinical pathways relating to those medical conditions to form a merged clinical pathway. This preliminary process may comprise: comparing the care steps and step order in one of the predefined clinical pathways with the care steps and step order in one or more other predefined clinical pathways; identifying any care steps in the one predefined clinical pathway which match care steps and step order in one or more of the other predefined clinical pathways; selecting from any unmatched care steps in the predefined clinical pathways one or more care steps which satisfies most appropriately requirements for the merged clinical pathway which take into account the matched care steps, step order and information associated with the unmatched care step(s); and forming the merged clinical pathway from the matching care steps and the selected care step(s).

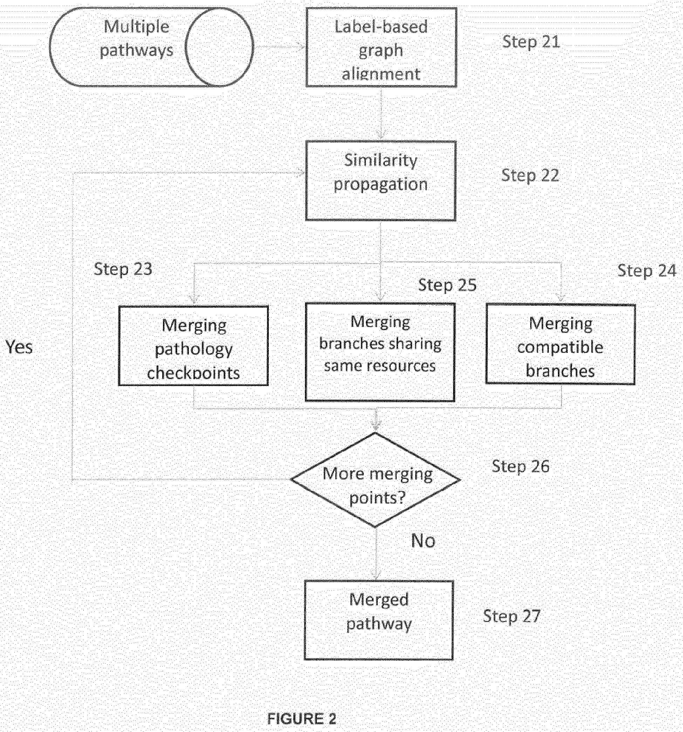

Multiple clinical pathways can be integrated in different ways. The present application document describes, with reference to the flowchart of Figure 2, an approach based on multiple strategies, in which clinical pathways are considered as graphs, with tasks (care steps or caring functions) as graph vertices where the interconnecting paths between vertices indicate the sequence or flow of processes. That is, the clinical pathways are considered as labelled, directed graphs. However, other approaches, which also embody the present invention, may be used, such as applying methods from constraint satisfaction problems, or machine learning methods, for example.

Before carrying out the enhancement method of the present invention, in an embodiment of the present invention each predefined clinical pathway is modelled as a graph having labelled vertices respectively representing different care steps in the clinical pathway and interconnecting lines between the vertices representing the flow of the pathway.

Initial alignment candidates are suggested based on graph characteristics (Step 21). For example, labels of pathway vertices can be used to find converging points where multiple pathways contain care steps in common. For instance, if two pathways suggest a histopathology check, this can be a care step where the two pathways should be merged.

In Step 22 of Figure 2, "Similarity propagation", it is considered how known similarities affect other unmatched care steps. Many existing string and graph based alignment technologies can be applied. The most basic one can be simple string distance computation based on the label strings. This can be enhanced with graph edit distance to penalise the initial string distance based suggestions. For instance, assume there are two pathways: *A*→ *B*→ *C*₁→ *D*→ *C*₂→*E* and *A* → *C* → *E*. After matching the "*A*" and "*E*" care steps, two candidate care steps could be aligned with "*C*", namely *C*₁ and *C*₂. The graph characteristics suggest that "*C*" has strong influence from *"E".* Therefore, "*C*₂" will be considered to be more appropriate than "*C*₁" with respect to "*C*". This is based on the assumption that *"C*₂ → *E"* shows a stronger structural similarity than "*A* → ... →*C*₁" due to the existence of "*B*" step in the latter case. However, a second level of screening is also needed before ignoring or skipping *B* → *C*₁, e.g. test results from A demand that B is mandatory. So in this case, another factor such as test results or biological readings/sensor data would also be considered before considering the most appropriate path to be followed next.

When merging clinical pathways, it is advantageous to consider domain knowledge. For example, candidate pathway vertices may be subject to further prioritisation based on the following aspects:
1. Pathology checkpoints with significant risk for patients (Step 23)
2. Pathology checkpoints with high cost for clinical centres (Step 25)
3. The crossover between multiple disciplines: e.g. the triple assessment of breast cancer (Step 24)

In order to utilise such information, quantification measures are necessary. For risk assessment, different levels of significance can be the basis for quantification. For instance, risk of immediate death can be quantified by a number between 7-10 with very high death risk corresponding to 10, and low death risk corresponding to 7. Alternatively, the quantification can be done based on statistics from a cohort of patients sharing the same characteristics. Other suitable quantification techniques are described, for example, in EP2677445A and EP2685407A.

Cost for clinical centres can be easily obtained based on the cost of equipment and cost of personnel Vertices with high expected costs should be given higher priority when aligning. Merging such tests (for joint assessments) where appropriate can remove or lessen the chance of performing duplicated tests with high cost.
Emphasis can also be given to caring functions that involve multiple different domains. For instance, if a caring function A involves multiple experts while a caring function B only summons up those from the same department, higher preference should be given to A.

After these factors are taken into account and there are no more merging points to be added (Step 26), and the steps which are to be included in the merged pathway have been chosen, a single clinical pathway is formulated (Step 27).

Clinical pathways are not isolated from administrative and operational processes in a clinical centre. In many cases, the success of clinical pathway execution is highly dependent on non-clinical processes. As shown in Step 2 of the method of Figure 1, the clinical pathway (predefined or merged) is integrated with at least one non-clinical process so as to associate non-clinical data from the non-clinical process with at least one care step which is related to that non-clinical data. The non-clinical data associated with the care step is used to produce one or more enriched clinical pathways. Such enhancement of the clinical pathway can help a clinician make better informed decisions on patient care.

The non-clinical data may relate to administrative and/or clinical procedures (such as requirements for authorisation, timing, personnel, cost, and/or equipment) and/or clinical assets of one or more clinical centres (such as staff resources, facilities, location, and/or equipment resources). One or more predefined criteria of care steps in the enriched clinical pathway are preferably aligned with one or more predefined criteria of related non-clinical steps in the non-clinical process

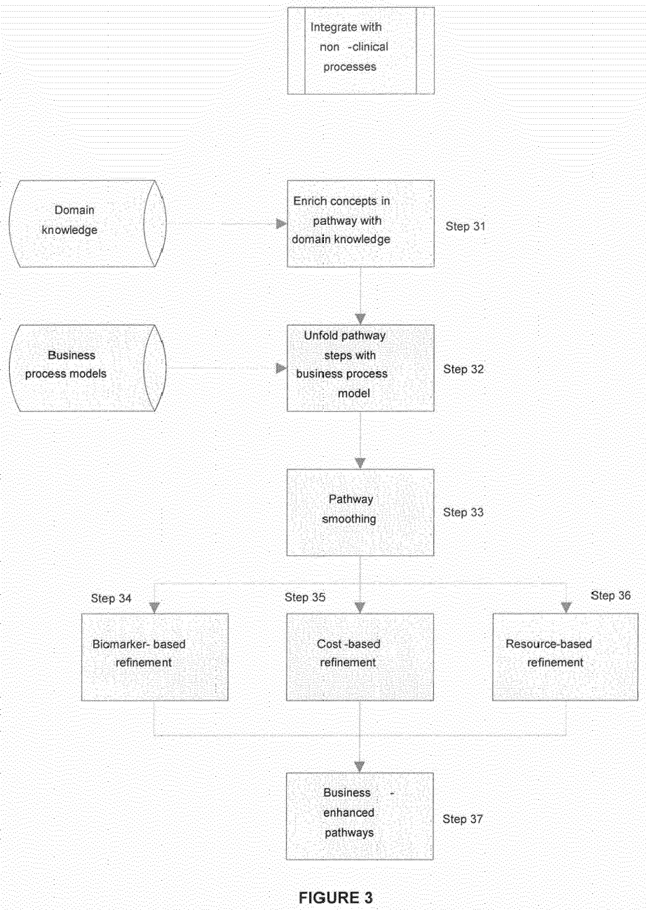

By way of example, and with reference to the flowchart of Figure 3, clinical and non-clinical processes may be integrated/aligned as follows:
Firstly a domain knowledge model *Oₐₛₛₑₜ* is constructed that represents the key clinical and administrative assets of a clinical centre. Such assets may include:
   clinical equipment and diagnostic instruments, clinical materials, infrastructure (e.g. water supply, electric power supply, compressed gas supply, etc.). It may also include the clinical staff resources such as nurses, doctors, radiologists, etc. Information that is preferably also included in such a domain model includes:
      location, authorisation channel, operating team, etc. Ideally, such knowledge model(s) should be represented in a computer understandable language to facilitate automated reasoning. In the following explanation we assume that all the business processes are already digitised to be used by computers.

The domain ontology *Oₐₛₛₑₜ* can be used in ontology-based process alignment as follows:
Step 31 - Enrich clinical step with the domain ontology: if a step of the clinical pathway refers to a particular medical diagnostic method, the ontology can be consulted to extend this step with related knowledge from the ontology. This is done by querying the individual hospital-based *Oₐₛₛₑₜ* to retrieve all the instances that related to the subject step. For instance if a lower limb computed tomography angiography (CTA) needs to be carried out, the system should first retrieve all the CTA equipment instances from *Oₐₛₛₑₜ*, traverse queries will then be used to retrieve all the related instances of the CTA instances from *Oₐₛₛₑₜ*. All this is cached in *I*_{*CTA*-}*_{LL}.*

Step 32 - For each different hospital department from *I*_{*CTA*-*LL*}, the corresponding business processes will be retrieved. Time constraints may be taken into account when integrating clinical pathways and business processes. In this case the integration will not be used to enrich a single pathway step. Instead, the integration serves to ensure that different steps from both clinical pathways and business processes are carried out along parallel timelines. For instance, assume *A → B → C* → *D* is a clinical pathway where *C* prompts a CTA to be carried out, and *O* → *P* → *Q* → *R* is a business process for acquiring and authorising a CTA from one of the available locations where *Q* indicates the approval of a referral and *R* the initial preparation for CTA. The integration process will enrich *C* with data from the business process. However, instead of expanding step *C,* a time constraint satisfaction algorithm will be used to parallelise steps before *C,* i.e. *A → B* with *O* → *P,* and steps following *C,* i.e. *D,* with *R*.

Step 33 - The result may be multiple enriched clinical pathways (for instance due to the existence of multiple CTA sites). In this case the system may evaluate the overall length of expected time period for each of the multiple enriched pathways and give higher preference to the pathway with the shortest overall duration of the parallelised pathway and business processes.

Steps 34, 35 and 36 - These multiple enriched clinical pathway instances may then be subject to further refinement/re-sorting, based for example on resource/equipment availability, distance from the patient's location (if it is an inpatient procedure), cost of running, personnel availability, etc.

Step 37 - Clinical pathways enhanced (enriched) with data from non-clinical/business processes are thereby obtained.

Enriched clinical pathways may be further refined to clearly define one or more patient caring milestones, as shown in Step 3 of Figure 1. That is, in an embodiment of the present invention, it is determined, according to one or more predefined refinement conditions, whether to split or merge one or more steps of the enriched clinical pathway. If so the step(s) is split or merged to produce a refined enriched clinical pathway. For example, steps in two or more independent pathways may be merged to provide a common checkpoint across several pathways. Alternatively, it may be desirable to split a step into several sequential steps to provide a more detailed checkpoint in the pathway.

The refinement conditions may comprise at least one of:
(1) an expected positive or negative change in the patient's biological markers (biomarkers) at that care step beyond a first predetermined threshold - for example a change in the prognosis, expected change of vital signs, etc. For instance, if a particular type of chemotherapy is expected to make significant change to a patient's biomarkers, it should be separate from others as an independent clinical pathway step. If in the original step multiple types of drugs are prescribed with different impacts on patient's biomarkers and vital signs, they should be split into different steps. A threshold can be predefined by the users. For instance, if a step can change the expected five year survival rate from 93% to 95% and the threshold is set to 0.5%, this step should become a single step in the clinical pathway;
(2) an expected positive or negative change in the financial cost of the care step beyond a second predetermined threshold; and
(3) a requirement for that care step to involve more than one clinical centre.

The clinical pathways, merged, enriched and refined through the above processes, can be used to treat patients without further alteration. However, there may still be more than one enriched clinical pathway suitable for treating a patient, and the decision as to which clinical pathway to use can change during execution of a clinical pathway depending upon on the changing condition of each individual patient. In an embodiment of the present invention therefore, as shown in Step 4 of Figure 1, status conditions indicative of the current status of the patient are used to dynamically select a preferred clinical pathway, from the enriched clinical pathways, which best meets one or more preset criteria. The preferred clinical pathway may be selected by identifying a set of candidate care steps from all the care steps in the enriched clinical pathways and ranking the candidate steps in the set in accordance with one or more preset criteria comprising, for example, at least one of: degree of similarity between the current patient status and the input conditions of the care step; likely output conditions of the care step; and possible prognosis as a result of the care step. The candidate care steps may be identified by comparing input conditions of each care step with a patient's current status.

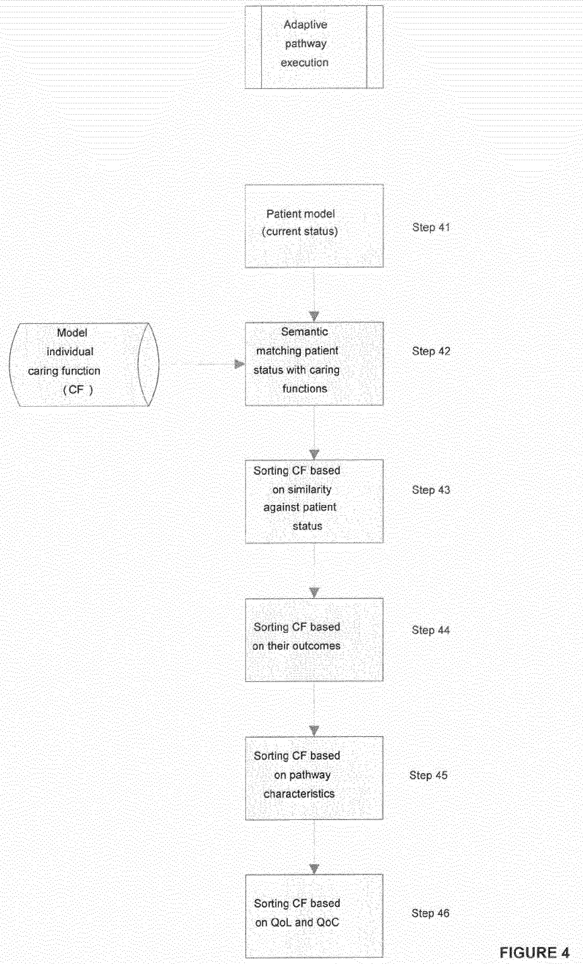

An example of a process for dynamically selecting a clinical pathway will be described below with reference to the flowchart of Figure 4. For each clinical caring function/care step in the clinical pathways, the input and output conditions are modelled. The input conditions may include a patient's biomarkers, vital sign readings, cost, time constraints (availability), physical constraints (e.g. constraints on mobility), etc. The output conditions may include the expected improvement or deterioration of a patient's biomarkers, vital sign readings, cost, as well as effectiveness and quality of care (for example as proposed in EP2677445A and EP2685407A.

Step 41 - The patient's current status is modelled (including biomarkers, vital signs, time and physical constraints, medical histories, allergic reactions, etc.).

Step 42 - The patient's current status is matched against the input conditions of each caring function. This filters out a set of candidate caring functions.

The resultant set of candidate caring functions is then sorted based on:
a. Similarity between the patient's status and the input conditions of each caring function (Step 43)
b. Quantitative comparison of the likely output conditions of each caring function (Step 44)
c. Prognosis of caring functions: this is done by computing a weighted distance between the selected caring function and the termination point of the clinical pathway (Step 45).
d. When there are multiple termination points, a ranking mechanism can be used to select the one giving patients the best quality of care (QoC) and quality of life (QoL) (Step 46). Quantification of such quality measures can be obtained, for example, using the techniques proposed in EP2677445A and EP2685407A. Follow-up caring functions may also be automatically suggested.

Accordingly, with each significant change of a patient's status or the resources available for treating that patient, the clinical pathway selected for the patient by the method may change to a lesser or greater extent, effectively dynamically adapting the current clinical pathway to reflect the current status of both the patient and the clinical centre(s). Such an approach provides more flexibility than a single, rigid pathway.

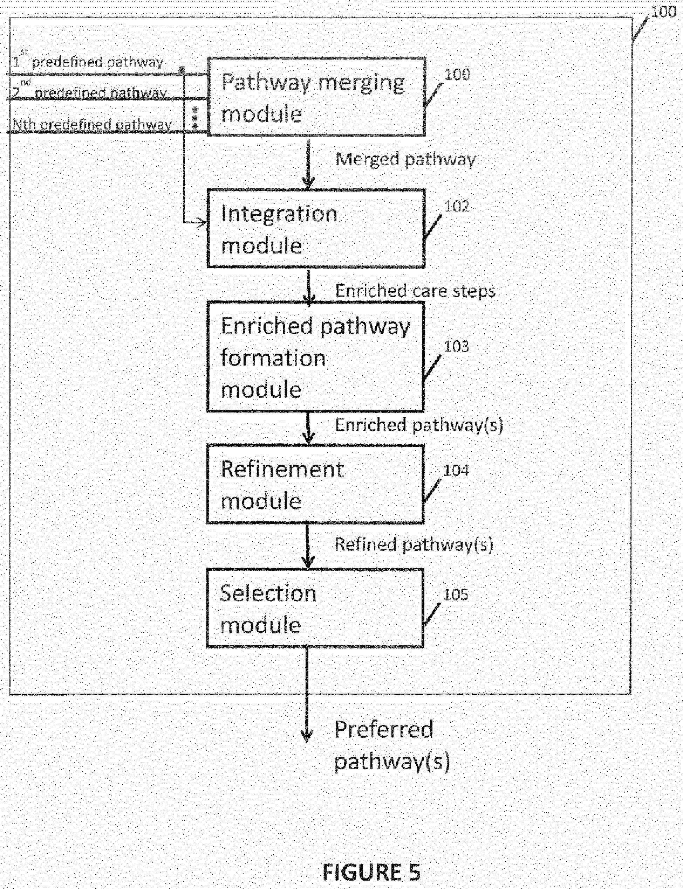

In summary, an embodiment of the present invention can enhance clinical pathways by integrating non-clinical data from related non-clinical processes into the clinical pathway to allow a clinician to make better decisions regarding a patient's treatment on the basis of the enriched pathways. Where a patient has more than one disorder to be treated, in an initial step multiple clinical pathways may be simplified by merging the pathways into one. Enriched pathways may be refined to define clear milestones and checkpoints to avoid delay in treatment. In addition, a pathway may be changed so as to dynamically adapt the care steps to the individual situation of each patient, thereby potentially shortening the course of caring and potentially reducing the cost of caring. As illustrated in Figure 5, computer apparatus 100 configured to carry out a clinical pathway enhancement method embodying the present invention may comprise: pathway merging module 101 configured to merge, if necessary, two or more predefined clinical pathways to form a merged clinical pathway for enhancement in the clinical pathway enhancement method; integration module 102 configured to integrate the clinical pathway (the merged clinical pathway or a predefined clinical pathway, as appropriate) with at least one non-clinical process by associating non-clinical data from the non-clinical process with at least one care step which is related to that non-clinical data; enriched pathway formation module 103 configured to use the non-clinical data associated with the care step to produce one or more enriched clinical pathways and align the timing of care steps in the enriched clinical pathway with the timing of related non-clinical steps in the non-clinical process; refinement module 104 configured to determine, according to one or more predefined refinement conditions, whether to split the enriched clinical pathway at one or more care steps in that clinical pathway, and, if so determined, to split the clinical pathway at the care step(s) to produce two or more refined enriched clinical pathways; and selection module 105 operable, where the total number of enriched clinical pathways is greater than one, to use status conditions indicative of the current status of the patient to dynamically select a preferred clinical pathway, from the enriched clinical pathways, which best meets one or more preset criteria, by identifying a set of candidate care steps from all the care steps in the enriched clinical pathways and ranking the candidate steps in the set in accordance with one or more preset criteria.

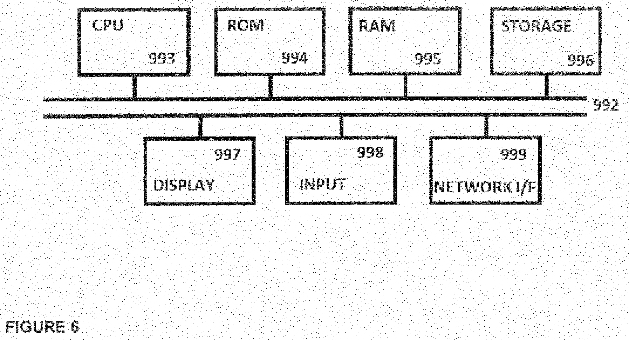

Figure 6 is a block diagram of a computing device, such as a data storage server, which embodies the present invention, and which may be used to implement a method of an embodiment and perform the tasks of apparatus of an embodiment. The computing device comprises a computer processing unit (CPU) 993, memory, such as Random Access Memory (RAM) 995, and storage, such as a hard disk, 996. Optionally, the computing device also includes a network interface 999 for communication with other such computing devices of embodiments. For example, an embodiment may be composed of a network of such computing devices. Optionally, the computing device also includes Read Only Memory 994, one or more input mechanisms such as keyboard and mouse 998, and a display unit such as one or more monitors 997. The components are connectable to one another via a bus 992.

The CPU 993 is configured to control the computing device and execute processing operations. The RAM 995 stores data being read and written by the CPU 993. The storage unit 996 may be, for example, a non-volatile storage unit, and is configured to store data.

The display unit 997 displays a representation of data stored by the computing device and displays a cursor and dialog boxes and screens enabling interaction between a user and the programs and data stored on the computing device. The input mechanisms 998 enable a user to input data and instructions to the computing device.

The network interface (network I/F) 999 is connected to a network, such as the Internet, and is connectable to other such computing devices via the network. The network I/F 999 controls data input/output from/to other apparatus via the network. Other peripheral devices such as microphone, speakers, printer, power supply unit, fan, case, scanner, trackerball etc may be included in the computing device.

Methods embodying the present invention may be carried out on a computing device such as that illustrated in Figure 6. Such a computing device need not have every component illustrated in Figure 6, and may be composed of a subset of those components. A method embodying the present invention may be carried out by a single computing device in communication with one or more data storage servers via a network. The computing device may be a data storage itself storing at least a portion of the data. A method embodying the present invention may be carried out by a plurality of computing devices operating in cooperation with one another. One or more of the plurality of computing devices may be a data storage server storing at least a portion of the data.

Embodiments of the present invention may be implemented in hardware, or as software modules running on one or more processors, or on a combination thereof. That is, those skilled in the art will appreciate that a microprocessor or digital signal processor (DSP) may be used in practice to implement some or all of the functionality described above.

The invention may also be embodied as one or more device or apparatus programs (e.g. computer programs and computer program products) for carrying out part or all of the methods described herein. Such programs embodying the present invention may be stored on computer-readable media, or could, for example, be in the form of one or more signals. Such signals may be data signals downloadable from an Internet website, or provided on a carrier signal, or in any other form.

## Claims

1. Computer apparatus configured to carry out a clinical pathway enhancement method in respect of a clinical pathway having a plurality of care steps, where the clinical pathway is a predefined clinical pathway which is associated with an individual patient or a cohort of patients, or a merged clinical pathway obtained by merging two or more such predefined clinical pathways associated with the patient or patient cohort, the apparatus comprising:
integration means configured to integrate the clinical pathway with at least one non-clinical process, the integration means being operable to associate non-clinical data from the non-clinical process with at least one care step which is related to that non-clinical data; and
enriched pathway formation means configured to use the non-clinical data associated with the care step to produce one or more enriched clinical pathways.

2. Apparatus as claimed in claim 1, wherein the enriched pathway formation means are operable to align one or more predefined criteria of care steps in the enriched clinical pathway with one or more predefined criteria of related non-clinical steps in the non-clinical process.

3. Apparatus as claimed in claim 1 or 2, wherein the integration means are operable to associate with a care step non-clinical data relating to administrative and/or clinical procedures, and/or clinical assets of one or more clinical centres,
where the administrative and/or clinical procedures comprise at least one of:
authorisation requirements; timing requirements; personnel requirements; cost requirements; and equipment requirements; and
where the clinical assets comprise at least one of: staff resources; facilities; location; and equipment resources.

4. Apparatus as claimed in any one of claims 1 to 3, further comprising refinement means configured to determine, according to one or more predefined refinement conditions, whether to split or merge one or more care steps of the enriched clinical pathway, and, if so determined, to split or merge the care step(s) to produce a refined enriched clinical pathway.

5. Apparatus as claimed in claim 4, wherein the refinement conditions comprise at least one of: an expected positive or negative change in the patient's biological markers at that care step beyond a first predetermined threshold; an expected positive or negative change in the financial cost of the care step beyond a second predetermined threshold; and a requirement for that care step to involve more than one clinical centre.

6. Apparatus as claimed in any one of claims 1 to 5, further comprising selection means operable, where the total number of enriched clinical pathways is greater than one, to use status conditions indicative of the current status of the patient to dynamically select a preferred clinical pathway, from the enriched clinical pathways, which best meets one or more preset criteria.

7. Apparatus as claimed in claim 6, wherein the selection means are operable to select the preferred clinical pathway by identifying a set of candidate care steps from all the care steps in the enriched clinical pathways and ranking the candidate steps in the set in accordance with one or more preset criteria.

8. Apparatus as claimed in claim 7, wherein the selection means are operable to identify the candidate care steps by comparing input conditions of each care step with a patient's current status.

9. Apparatus as claimed in claim 7 or 8, wherein the one or more preset criteria for ranking the candidate steps comprises at least one of: degree of similarity between the current patient status and the input conditions of the care step; likely output conditions of the care step; and possible prognosis as a result of the care step.

10. Apparatus as claimed in any one of claims 1 to 9, operable, before carrying out the enrichment method, to model the or each predefined clinical pathway as a graph having labelled vertices respectively representing different care steps in the clinical pathway and interconnecting lines between the vertices representing the flow of the pathway, wherein the graph is subsequently used in the enrichment method.

11. Apparatus as claimed in any one of claims 1 to 10, further comprising pathway merging means configured to merge predefined clinical pathways to form a merged clinical pathway for enrichment in the clinical pathway enhancement method, which pathway merging means are operable to:
compare the care steps and step order in one of the predefined clinical pathways with the care steps and step order in one or more other predefined clinical pathways;
identify any care steps in the one predefined clinical pathway which match care steps and step order in one or more of the other predefined clinical pathways;
select from any unmatched care steps in the predefined clinical pathways one or more care steps which satisfies most appropriately requirements for the merged clinical pathway which take into account the matched care steps, step order and information associated with the unmatched care step(s); and
form the merged clinical pathway from the matching care steps and the selected care step(s).

12. A computer-implemented clinical pathway enhancement method for enhancing a clinical pathway having a plurality of care steps, where the clinical pathway is a predefined clinical pathway which is associated with an individual patient or a cohort of patients, or a merged clinical pathway obtained by merging two or more such predefined clinical pathways associated with the patient or patient cohort, the method comprising:
integrating the clinical pathway with at least one non-clinical process so as to associate non-clinical data from the non-clinical process with at least one care step which is related to that non-clinical data; and
using the non-clinical data associated with the care step to produce one or more enriched clinical pathways.

13. A method as claimed in claim 12, further comprising determining, according to one or more predefined refinement conditions, whether to split or merge one or more care steps of the enriched clinical pathway, and, if so determined, splitting or merging the care step(s) to produce a refined enriched clinical pathway.

14. A method as claimed in claim 12 or 13, further comprising using status conditions indicative of the current status of the patient to dynamically select a preferred clinical pathway, where the total number of enriched clinical pathways is greater than one, which best meets one or more preset criteria.

15. A computer program which, when run on a computer, causes that computer to carry out the method of any one of claims 12 to 14.
